# EUROPEAN PATENT SPECIFICATION

(11) **EP 4 581 007 B1**
(45) Date of publication and mention of the grant of the patent: **27.05.2026**
(21) Application number: 23764852.2
(22) Date of filing: 30.08.2023
(51) Int. Cl.: C07C 49/557, C07C 69/753, C11B 9/00

(54) **DIMETHYLBICYCLOHEPTENYL AND DIMETHYLBICYCLOHEPTANYL DERIVATIVES AND THEIR USE AS ODORANTS**
DIMETHYLBICYCLOHEPTENYL- UND DIMETHYLBICYCLOHEPTANYLDERIVATE UND IHRE VERWENDUNG ALS RIECHSTOFFE
DÉRIVÉS DE DIMÉTHYLBICYCLOHEPTÉNYLE ET DE DIMÉTHYLBICYCLOHEPTANYLE ET LEUR UTILISATION COMME SUBSTANCES ODORANTES

(30) Priority: 01.09.2022 WO PCT/CN2022/116531
(43) Date of publication of application: 09.07.2025
(73) Proprietor: Givaudan SA, 1214 Vernier (CH)
(72) Inventor: ZHOU, Lijun, Shanghai 201203 (CN); GOEKE, Andreas, 8310 Kemptthal (CH)
(74) Representative: Global Patents
(86) International application number: PCT/EP2023/073762
(87) International publication number: WO 2024/047093

(56) References cited:
- EP-B1- 0 746 552
- US-A- 4 147 672

## Description

### TECHNICAL FIELD

The present invention is concerned with flavor and fragrance ingredients, and to compositions containing them that are useful in flavor and fragrance applications. It furthermore relates to a method of making them. In particular, the present invention is concerned with dimethylbicycloheptenyl and dimethylbicycloheptanyl derivatives of formula (I) possessing green-galbanum, and fruity-pineapple odor notes.

### BACKGROUND

In the flavor and fragrance industry, perfumers and flavorists are continually looking for new compounds possessing unique olfactory properties. For example, there is a need for ingredients that are suitable for the flavor and fragrance industry possessing green fruity-pineapple odor notes reminiscent of galbanum.

Galbanum oil is a natural perfuming ingredient highly appreciated by perfumers for its green character. However, its use is limited by a high cost. Synthetic replacers having an odor reminiscent of galbanum oil are described, for example, in US 4147672 disclosing 3,3-dimethyl-cyclohecyl and 3,3-dimethyl-cyclohexenyl derivatives. Fragrance ingredients reminiscent of galbanum oil are also described in EP 913383 disclosing spirocyclic compounds, or WO2012110281 disclosing 1-(5-ethyl-5-methyl-1-cyclohexen-1-yl)-4-penten-1-one imparting odor notes of the galbanum type.

Galbanum is valued for its ability to impart a rich, spicy green scent. The green galbanum odor family is quite important for the creation of fragrance accords. Some famous fragrances get their character from this odor family.

There still remains a need for new fragrance ingredients reminiscent of galbanum. In addition to the primary objection to provide new fragrance ingredients of the galbanum odor family, the ingredients should be preferably being derivable from renewable resources. The use of renewable starting material is becoming more and more significant because of the increasing market demand for new fragrances based on renewable raw materials and hence at least partial constituting a replacement of ingredients obtained from petrochemical raw materials.

Surprisingly inventors found a new class of ingredients of formula (I) as defined herein below possessing the highly sought-after green-galbanum, and fruity-pineapple odor notes, all of which are derivable from renewable starting materials.

### SUMMARY

In accordance with a first aspect of the present invention there are provided compounds of formula (I) in the form of any one of its stereoisomers or mixture thereof 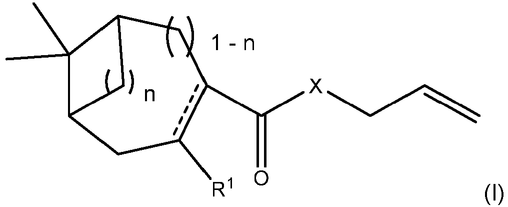 wherein
the dotted line together with the carbon-carbon bond forms a single bond or a double bond, n is 1 or 0;
X is selected from -O- and -CH₂-, and
R' is hydrogen or methyl.

In accordance with a second aspect of the present invention there is provided a fragranced article comprising as odorant a compound of formula (I) of the first aspect.

In accordance with a third aspect of the present invention there is provided a method of improving, enhancing and/or modifying a consumer product base or consumable product base by means of adding thereto an olfactory acceptable amount of a compound for formula (I) of the first aspect.

In accordance with a fourth aspect of the present invention there is provided a flavored product comprising a compound of formula (I) of the first aspect.

In accordance with a fifth aspect of the present invention there is provided a fragrance or flavor composition comprising a compound of formula (I) of the first aspect.

In accordance with a sixth aspect of the present invention there is provided the use as flavor or fragrance of a compound of formula (I) of the first aspect.

Certain embodiments of any aspect of the present invention may provide one or more of the following advantages:
- green-galbanum, and fruity-pineapple odor profiles,
- powerful compounds,
- made from natural and/or renewable resources, and
- biodegradable.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that dimethylbicycloheptenyl derivatives possessing green-galbanum, and fruity-pineapple odor notes.

In a first aspect of the invention, there is provided a compound of formula (I) 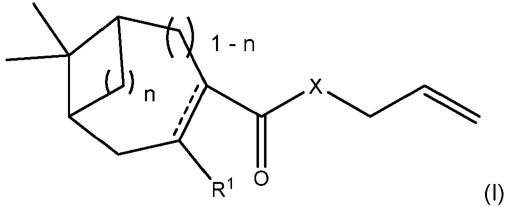 wherein
the dotted line together with the carbon-carbon bond forms a single bond or a double bond; n is 1 or 0;
X is selected from -O- and -CH₂-; and
R' is hydrogen or methyl.

In one particular embodiment there is provided a compound of formula (I) wherein n is 1, i.e. a compound of formula (la) 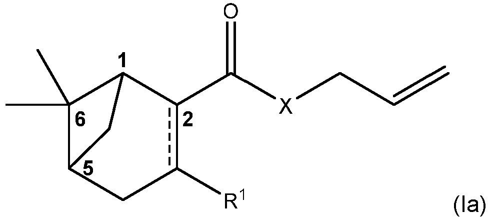 wherein
the dotted line together with the carbon-carbon bond forms a single bond or a double bond; X is selected from -O- and -CH₂-; and
R' is hydrogen or methyl.

In another particular embodiment there is provided a compound of formula (la) wherein R¹ is hydrogen.

In another particular embodiment there is provided a compound of formula (I) wherein n is 0, i.e. a compound of formula (Ib) 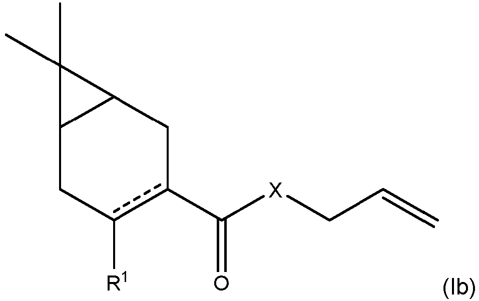
the dotted line together with the carbon-carbon bond forms a single bond or a double bond; X is selected from -O- and -CH₂-; and
R' is hydrogen or methyl.

In another particular embodiment there is provided a compound of formula (Ib) wherein X is -CH₂-.

In another particular embodiment the compound of formula (I) is selected from the group consisting of 1-(6,6-dimethylbicyclo[3.1.1]hept-2-en-2-yl)pent-4-en-1-one, 1-(6,6-dimethylbicyclo[3.1.1]heptan-2-yl)pent-4-en-1-one, allyl 6,6-dimethylbicyclo[3.1.1]hept-2-ene-2-carboxylate, 1-(7,7-dimethylbicyclo[4.1.0]hept-3-en-3-yl)pent-4-en-1-one, 1-(4,7,7-trimethylbicyclo[4.1.0]hept-3-en-3-yl)pent-4-en-1-one, and allyl 7,7-dimethylbicyclo[4.1.0]hept-3-ene-3-carboxylate.

The compounds of formula (I) comprise two or more chiral centers and as such may exist as a mixture of stereoisomers, or they may be resolved as isomerically pure forms. If it is desired to prepare individual stereoisomers, this may be achieved according to methodology known in the art, e.g. preparative HPLC and GC or by stereoselective syntheses, or simply by using as starting material the desired isomerically pure form, which are available from natural resources.

In one particular embodiment the compounds of formula (Ia) wherein the dotted line together with the carbon-carbon bond forms a single bond are compounds wherein the relative configuration of the ring system is (1S,5R), for example, 1-((1S,5R)-(6,6-dimethylbicyclo[3.1.1]hept-2-en-2-yl)pent-4-en-1-one.

In another particular embodiment the compounds of formula (Ia) wherein the dotted line together with the carbon-carbon bond forms a single bond are compounds wherein the relative confirguration of the ring system is (1R,5S), for example, allyl (1R,5S)-6,6-dimethylbicyclo[3.1.1]hept-2-ene-2-carboxylate.

In another particular embodiment the compounds of formula (Ia) wherein the dotted line together with the carbon-carbon bond forms a double bond are compounds wherein the relative configuration of the ring system is (1S,5S), for example, 1-((1S,5S)-6,6-dimethylbicyclo[3.1.1]heptan-2-yl)pent-4-en-1-one.

The compounds of formula (I) may be used alone, as isomeric mixture thereof, or in combination with known odorant molecules selected from the extensive range of natural products, and synthetic molecules currently available, such as essential oils, alcohols, aldehydes and ketones, ethers and acetals, esters and lactones, macrocycles and heterocycles, and/or in a mixture with one or more ingredients or excipients conventionally used in conjunction with odorants in fragrance compositions, for example, carrier materials, and other auxiliary agents commonly used in the art.

As used herein, "carrier material" means a material which is practically neutral from an odorant point of view, i.e. a material that does not significantly alter the organoleptic properties of odorants.

The term "auxiliary agent" refers to ingredients that might be employed in a fragrance composition for reasons not specifically related to the olfactive performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a fragrance composition. A detailed description of the nature and type of adjuvants commonly used in fragrance compositions containing same cannot be exhaustive, but it has to be mentioned that said ingredients are well known to a person skilled in the art.

As used herein, 'fragrance composition' means any composition comprising a compound of formula (I), or a mixture thereof and a base material, e.g. a diluent conventionally used in conjunction with odorants, such as diethyl phthalate (DEP), dipropylene glycol (DPG), isopropyl myristate (IPM), pentane-1,2-diol, triethyl citrate (TEC) and alcohol (e.g. ethanol). Optionally, the composition may comprise an anti-oxidant adjuvant. Said anti-oxidant may be selected from Tinogard^{®} TT (BASF), Tinogard^{®} Q (BASF), Tocopherol (including its isomers, CAS 59-02-9; 364-49-8; 18920-62-2; 121854-78-2), 2,6-bis(1,1-dimethylethyl)-4-methylphenol (BHT, CAS 128-37-0) and related phenols, hydroquinones (CAS 121-31-9).

The following list comprises examples of known odorant molecules, which may be combined with a compound of formula (I), or a mixture thereof:
- essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, lavender oil and/ or ylang-ylang oil;
- alcohols, e.g. cinnamic alcohol ((E)-3-phenylprop-2-en-1-ol); cis-3-hexenol ((Z)-hex-3-en-1-ol); citronellol (3,7-dimethyloct-6-en-1-ol); dihydro myrcenol (2,6-dimethyloct-7-en-2-ol); Ebanol^{™} ((E)-3-methyl-5-(2,2,3-trimethylcyclopent-3-en-1-yl)pent-4-en-2-ol); eugenol (4-allyl-2-methoxyphenol); ethyl linalool ((E)-3,7-dimethylnona-1,6-dien-3-ol); farnesol ((2E,6Z)-3,7,11-trimethyldodeca-2,6,10-trien-1-ol); geraniol ((E)-3,7-dimethylocta-2,6-dien-1-ol); Super Muguet^{™} ((E)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); Rhodinol^{™} (3,7-dimethyloct-6-en-1-ol); Sandalore^{™} (3-methyl-5-(2,2,3-trimethylcyclopent-3-en-1-yl)pentan-2-ol); terpineol (2-(4-methylcyclohex-3-en-1-yl)propan-2-ol); or Timberol^{™} (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;
- aldehydes and ketones, e.g. anisaldehyde (4-methoxybenzaldehyde); alpha amyl cinnamic aldehyde (2-benzylideneheptanal); Georgywood^{™} (1-(1,2,8,8-tetramethyl-1,2,3,4,5,6,7,8-octahydronaphthalen-2-yl)ethanone); Hydroxycitronellal (7-hydroxy-3,7-dimethyloctanal); Iso E Super^{®} (1-(2,3,8,8-tetramethyl-1,2,3,4,5,6,7,8-octahydronaphthalen-2-yl)ethanone); Isoraldeine^{®} ((E)-3-methyl-4-(2,6,6-trimethylcyclohex-2-en-1-yl)but-3-en-2-one); Hedione^{™} (methyl 3-oxo-2-pentylcyclopentaneacetate); 3-(4-isobutyl-2-methylphenyl)propanal; maltol; methyl cedryl ketone; methylionone; verbenone; and/or vanillin;

- ether and acetals, e.g. Ambrox^{®} (3a,6,6,9a-tetramethyl-2,4,5,5a,7,8,9,9b-octahydro-1H-benzo[e][1]benzofuran); geranyl methyl ether ((2E)-1-methoxy-3,7-dimethylocta-2,6-diene); and/ or Spirambrene^{®} (2',2',3,7,7-pentamethylspiro[bicyclo[4.1.0]heptane-2,5'-[1,3]dioxane]) ;
- esters and lactones, e.g. benzyl acetate; cedryl acetate ((1S,6R,8aR)-1,4,4,6-tetramethyloctahydro-1H-5,8a-methanoazulen-6-yl acetate); delta-decalactone (6-pentyltetrahydro-2H-pyran-2-one); Helvetolide^{®} (2-(1-(3,3-dimethylcyclohexyl)ethoxy)-2-methylpropyl propionate); delta-undecalactone (5-heptyloxolan-2-one); and / or vetiveryl acetate ((4,8-dimethyl-2-propan-2-ylidene-3,3a,4,5,6,8a-hexahydro-1H-azulen-6-yl) acetate);
- macrocycles, e.g. Ambrettolide ((Z)-oxacycloheptadec-10-en-2-one); ethylene brassylate (1,4-dioxacycloheptadecane-5,17-dione); and / or Exaltolide^{®} (16-oxacyclohexadecan-1-one); and
- heterocycles, e.g. isobutylquinoline (2-isobutylquinoline).

Thus there is provided in a further aspect of the invention a fragrance composition comprising a compound of formula (I).

The compounds of formula (I) may be used in a broad range of fragranced articles, e.g. in any field of fine and functional perfumery, such as perfumes, air care products, household products, laundry products, body care products and cosmetics. The compound can be employed in widely varying amounts, depending upon the specific article and on the nature and quantity of other odorant ingredients. The proportion is typically from 0.00001 to 3 weight per cent of the article. In one embodiment, the compound may be employed in a fabric softener in an amount from 0.0001 to 3 weight per cent (e.g. 0.001 to 1 including 0.5, 0.3, and 0.05 weight %). In another embodiment, the compound may be used in fine perfumery in amounts from 0.01 to 30 weight per cent (e.g. up to about 10 or up to 20 weight per cent), more preferably between 0.01 and 5 weight per cent (e.g. 0.01 to 0.1 weight per cent). However, these values are given only by way of example, since the experienced perfumer may also achieve effects or may create novel accords with lower or higher concentrations.

The compounds of formula (I) may be employed in a consumer product base simply by directly mixing the compound, or a fragrance composition comprising a compound of formula (I), or a mixture thereof, with the consumer product base, or it may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, and then mixed with the consumer product base.

Thus, the invention additionally provides a method of manufacturing a fragranced article, comprising the incorporation a compound of formula (I), or a mixture thereof as a fragrance ingredient, either by directly admixing to the consumer product base or by admixing a fragrance composition comprising a compound of formula (I), or a mixture thereof, which may then be mixed with a consumer product base, using conventional techniques and methods. Through the addition of an olfactory acceptable amount of a compound of formula (I), or a mixture thereof the odor notes of a consumer product base will be improved, enhanced, or modified.

Thus, the invention furthermore provides a method for improving, enhancing or modifying a consumer product base by means of the addition thereto of an olfactorily acceptable amount of a compound of formula (I), or a mixture thereof.

There is provided in a further aspect of the present invention a fragranced article comprising:
a) a compound of formula (I) 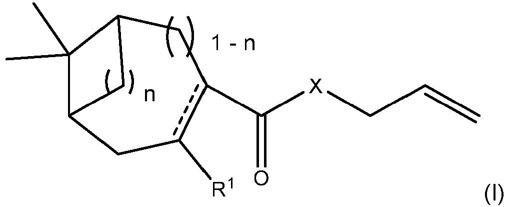 wherein
   the dotted line together with the carbon-carbon bond forms a single bond or a double bond,
   n is 1 or 0;
   X is selected from -O- and -CH₂-, and
   R' is hydrogen or methyl;
   and
b) a consumer product base.

As used herein, 'consumer product base' means a composition for use as a consumer product to fulfill specific actions, such as cleaning, softening, and caring or the like. Examples of such products include fine perfumery, e.g. perfume and eau de toilette; fabric care, household products and personal care products such as cosmetics, laundry care detergents, rinse conditioner, personal cleansing composition, detergent for dishwasher, surface cleaner; laundry products, e.g. softener, bleach, detergent; body-care products, e.g. shampoo, shower gel; air care products (includes products that contain preferably volatile and usually pleasant-smelling compounds which advantageously can even in very small amounts mask unpleasant odors). Air fresheners for living areas contain, in particular, natural and synthetic essential oils such as pine needle oils, citrus oil, eucalyptus oil, lavender oil, and the like, in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants.

Cosmetic products include:
(a) cosmetic skincare products, especially bath products, skin washing and cleansing products, skincare products, eye makeup, lip care products, nail care products, intimate care products, foot care products;
(b) cosmetic products with specific effects, especially sunscreens, tanning products, de-pigmenting products, deodorants, antiperspirants, hair removers, and shaving products;
(c) cosmetic dental-care products, especially dental and oral care products, tooth care products, cleaners for dental prostheses, adhesives for dental prostheses; and
(d) cosmetic hair care products, especially hair shampoos, hair care products, hair setting products, hair-shaping products, and hair coloring products.

This list of products is given by way of illustration, and is not to be regarded as being in any way limiting.

In one particular embodiment the consumer product base is selected form fine perfumery, and personal care products, including deodorants, hair care products, soaps, and the like. In one particular embodiment the consumer product base is a hair care product.

In a further particular embodiment the consumer product base is selected from fabric care products, including fabric softener, and home care products, including air fresheners, dish washers and the like.

The compounds of formula (I) wherein n is 1 and X is - CH₂- may, for example, be synthesized by oxidation of α-pinene to its corresponding aldehyde myrtenal following by Grignard reaction and then oxidation to get the corresponding ketone. Alternately, the compounds may, for example, be synthesized by oxidation of α-pinene to its corresponding aldehyde myrtenal, followed by hydrogenation, Grignard reaction and then oxidation to get the corresponding ketone.

The compounds of formula (I) wherein n is 1 and X is oxygen may, for example, further be synthesized by oxidation of α-pinene to its corresponding acid stepwisely, followed by esterification to the corresponding ester.

α-Pinene is found, for example, in the oils of many species of coniferous trees, notably in the pine. It exists in two enantiomers, (1S,5S)- or (-)-α-pinene and (1R,5R)- or (+)-α-pinene, both of which exist in nature. Whereas (-)-α-pinene is more common in European pines, (+)-α-pinene is more common in North America. The racemic mixture is also present in some oils, such as eucalyptus oil and orange peel oil.

The compounds of formula (I) wherein n is 0 may, for example, be synthesized by isomerization of 3-carene epoxide to 3(10)-caren-4-ol following by oxidation, Grignard reaction and then oxidation to get the corresponding ketone. Alternately, the compounds of formula (I) wherein n is 0 may, for example, be synthesized by isomerization of acetyl carene following by α-allylation to from the corresponding ketone.

The compounds of formula (I) wherein n is 0 and X is oxygen may, for example, be synthesized by isomerization of 3-carene epoxide to 3(10)-caren-4-ol following by oxidation to its corresponding acid, followed by Grignard reaction and oxidation to form the corresponding ester.

3-Carene epoxide is obtained via epoxidation from 3-carene (3,7,7-trimethylbicyclo[4.1.0]hept-3-ene), which is typically found, for example, in essential oils such as turpentine oil, juniper oil and pine needle oil. It exists in two enantiomers, (+)-3-carene or (1S,6R)-3-carene and (-)-3-carene or (1R,6S)-3-carene, both of which exist in nature.

The invention is now further described with reference to the following non-limiting examples. These examples are for the purpose of illustration only and it is understood that variations and modifications can be made by one skilled in the art.

### EXAMPLES

All reactions were performed under argon using solvents and reagents from commercial suppliers without further purification. Solvents for extraction and chromatography were technical grade and used without further purification. Flash chromatography was performed using Tsingdao Haiyang Chemical silica gel (200 - 300 mesh) and Santai Technologies silica flash columns. Unless otherwise noted, a mixture of Heptane: MTBE was used as eluent. NMR spectra were recorded with AW 400 MHz Bruker spectrometer instrument. The chemical shifts for ¹H NMR spectra was reported in δ (ppm) referenced to the residual proton signal of the deuterated solvent; coupling constants were expressed in Hertz (Hz). ¹³C NMR spectra were referenced to the carbon signals of the deuterated solvent. The following abbreviations are used: s = singlet, d = doublet, t = triplet, q = quartet, m = multiplet, dd = double doublet, bs = broad singlet. GC/MS spectral data were obtained from an Agilent 6890 N and MSD 5975 using a column HP-5 MS, 30 m, 0.25 mm, 0.25 µm.

### Example 1: 1-(6,6-Dimethylbicyclo[3.1.1]hept-2-en-2-yl)pent-4-en-1-one

a. To a solution of α-pinene (20.0 g, 147 mmol) and selenium dioxide (16.3 g, 147mmol) in 1,4-dioxane (100 mL) was slowly added acetic acid (13.2 g, 220 mmol) at 0°C and then stirred at 80°C for 16 h. The reaction was quenched by adding a saturated NH₄Cl solution, and the mixture was extracted with ethyl acetate (150 mL*3). The organic layer was washed with saturated NaHCO₃ solution, brine and dried over MgSO₄. Remove the solvent and the residue was distilled and gave myrtenal (6,6-dimethylbicyclo[3.1.1]hept-2-ene-2-carbaldehyde) (14.6 g, 97 mmol, 66% yield) as colorless liquid.
   ¹H NMR (400 MHz, CDCl₃) δ 9.44 (s, 1H), 6.75 - 6.69 (m, 1H), 2.87 (t, *J =* 5.5 Hz, 1H), 2.64 - 2.45 (m, 3H), 2.22 - 2.16 (m, 1H), 1.34 (s, 3H), 1.06 (d, *J =* 9.2 Hz, 1H), 0.75 (s, 3H) ppm. ¹³C NMR (101 MHz, CDCl₃) δ 191.2, 151.5, 147.8, 40.7, 38.1, 37.6, 33.0, 31.1, 25.7, 20.9 ppm. GC/MS (EI): m/z (%): 150 (1) [*M⁺*], 135 (14), 107 (74), 91 (40), 79 (100).
b. To a solution of but-3-en-1-ylmagnesium chloride (121 mL, 61mmol, 0.5 M) in tetrahydrofuran (100 mL) was added myrtenal (6,6-dimethylbicyclo[3.1.1]hept-2-ene-2-carbaldehyde, 7.0 g, 47 mmol) in Tetrahydrofuran (50 mL) at 10°C and stirred at room temperature for 3 h. The reaction was quenched by a saturated NH₄Cl solution, and extracted with ethyl ecetate (150 mL*3). The combined organic layer was washed with saturated NaHCO₃ solution and brine and dried over MgSO₄. Remove the solvent and the residue was purified via silica gel chromatography (PE:MTBE=4:1) and gave 1-(6,6-dimethylbicyclo[3.1.1]hept-2-en-2-yl)pent-4-en-1-ol (5.9 g, 29 mmol, yield: 61%) as light yellow liquid.
   ¹H NMR (400 MHz, CDCl₃,mixture of isomers) δ 5.96 - 5.67 (m, 1H), 5.48 - 5.39 (m, 1H), 5.10 - 4.89 (m, 2H), 4.07 - 3.97 (m, 1H), 2.48 - 2.00 (m, 7H), 1.61 - 1.30 (m, 6H), 1.17 - 1.12 (m, 1H), 0.90 - 0.77 (m, 3H) ppm. ¹³C NMR (101 MHz, CDCl₃, mixture of isomers) δ 150.5, 150.0, 138.6, 138.5, 118.1, 117.6, 114.7, 114.7, 74.4, 74.3, 42.1, 41.8, 41.0, 41.0, 37.8, 37.8, 33.9, 33.6, 31.8, 31.8, 31.2, 31.1, 30.2, 30.0, 26.2, 26.1, 21.4, 21.4 ppm. GC/MS (El): m/z (%): 206 (1) [*M⁺*]*,* 188 (2), 173 (4), 163 (24), 145 (55), 107 (99), 91 (100), 79 (77), 67 (88).
c. A mixture of 1-(6,6-dimethylbicyclo[3.1.1]hept-2-en-2-yl)pent-4-en-1-ol (4.0 g, 19.4 mmol), aluminium tri-sec-butoxide (1.4 g, 5.8 mmol) and acetic acid (0.1 g, 1.9 mmol) in MTBE (100 mL) was stirred at rt. for 20 min, then benzaldehyde (8.2 g, 78mmol) was added at r.t. and stirred for another 3 h. The reaction was quenched with water, and then extracted with MTBE (100 mL*3). The organic layer was dried over MgSO₄. Solvent was removed and the residue was purified via silica gel chromatography (PE:MTBE=4:1) to give 1-(6,6-dimethylbicyclo[3.1.1]hept-2-en-2-yl)pent-4-en-1-one (3.6 g, 18 mmol, yield: 91%) as colorless liquid.
   ¹H NMR (400 MHz, CDCl₃) δ 6.81 - 6.69 (m, 1H), 5.94 - 5.70 (m, 1H), 5.10 - 4.92 (m, 2H), 3.00 - 2.91 (m, 1H), 2.85 - 2.64 (m, 2H), 2.56 - 2.28 (m, 5H), 2.17 - 2.07 (m, 1H), 1.33 (s, 3H), 1.02 (d, *J =* 9.1 Hz, 1H), 0.74 (s, 3H) ppm. ¹³C NMR (101 MHz, CDCl₃) δ 198.4, 149.1, 137.6, 136.5, 115.0, 40.3, 39.6, 37.3, 36.1, 32.5, 31.1, 28.9, 25.9, 20.9 ppm. GC/MS (EI): m/z (%): 204 (1) [*M*⁺], 189 (3), 161 (27), 105 (100), 91 (25), 77 (22), 55 (48).

Odor description: green-galbanum, fruity-pineapple, metallic, fatty.

### Example 2: 1-((1R,5S)-6,6-Dimethylbicyclo[3.1.1]hept-2-en-2-yl)pent-4-en-1-one

a. (1*R*)-(-)-Myrtenal (50.0 g, 333 mmol) reacted with but-3-en-1-ylmagnesium chloride (433 mL, 433 mmol) according to the procedure in example 1 (step b) and get 1-((1*R*,5*S*)-6,6-dimethylbicyclo[3.1.1]hept-2-en-2-yl)pent-4-en-1-ol as colorless liquid (248 mmol, 51.1 g, 74% yield).
   GC/MS (EI): m/z (%): 206 (1) [*M⁺*], 188 (1), 173 (4), 163 (23), 145 (54), 107 (94), 91 (100), 79 (85), 67 (73), 55 (65).
b. 1-((1*R*,5*S*)-6,6-Dimethylbicyclo[3.1.1]hept-2-en-2-yl)pent-4-en-1-ol (40.0 g, 194 mmol) and benzaldehyde (82.0 g, 775 mmol) reacted according to the procedure in example 1 (step c) and gave 1-((1*R*,5*S*)-6,6-dimethylbicyclo[3.1.1]hept-2-en-2-yl)pent-4-en-1-one as a colorless liquid (142 mmol, 29.1 g, 74% yield).
   ¹H NMR (400 MHz, CDCl₃) δ 6.71 - 6.63 (m, 1H), 5.88 - 5.64 (m, 1H), 5.03 - 4.83 (m, 2H), 2.94 - 2.81 (m, 1H), 2.77 - 2.54 (m, 2H), 2.52 - 2.22 (m, 5H), 2.09 - 2.01 (m, 1H), 1.25 (s, 3H), 0.95 (d, *J =* 9.0 Hz, 1H), 0.67 (s, 3H) ppm. ¹³C NMR (101 MHz, CDCl₃) δ 198.5, 149.1, 137.6, 136.5, 115.0, 40.3, 39.6, 37.3, 36.1, 32.5, 31.1, 28.9, 25.9, 20.9 ppm. GC/MS (EI): m/z (%): 204 (1) [*M*⁺], 189 (3), 161 (26), 105 (100), 55 (55).

Odor description: green, galbanum, fruity, fatty, metallic, pineapple.

### Example 3: 1-((1S,5R)-6,6-dimethylbicyclo[3.1.1]hept-2-en-2-yl)pent-4-en-1-one

a. (1*S*)-(+)-Myrtenal ((1*S*,5*R*)-6,6-Dimethylbicyclo[3.1.1]hept-2-ene-2-carbaldehyde, 8.1 g, 54 mmol) reacted with but-3-en-1-ylmagnesium chloride (140 mL, 70 mmol) according to the procedure in example 1 (step b) and 1-((1S,5R)-6,6-dimethylbicyclo[3.1.1]hept-2-en-2-yl)pent-4-en-1-ol was obtained as colorless liquid (28 mmol, 5.7 g, 51% yield).
   ¹H NMR (400 MHz, CDCl₃, mixture of isomers) δ 5.93 - 5.72 (m, 1H), 5.52 - 5.33 (m, 1H), 5.09 - 4.87 (m, 2H), 4.03 - 3.97 (m, 1H), 2.48 - 2.01 (m, 7H), 1.62 - 1.41 (m, 3H), 1.32 - 1.28 (m, 3H), 1.18 - 1.12 (m, 1H), 0.87 - 0.80 (m, 3H) ppm. ¹³C NMR (101 MHz, CDCl₃, mixture of isomers) δ 150.5, 150.0, 138.6, 138.5, 118.0, 117.6, 114.7, 114.6, 74.4, 74.3, 42.1, 41.9, 41.0, 40.9, 37.8, 37.7, 33.9, 33.6, 31.9, 31.8, 31.2, 31.1, 30.2, 30.0, 26.2, 26.1, 21.5, 21.4 ppm. GC/MS (EI): m/z (%): 206 (1) [*M⁺*], 188 (3), 173 (3), 145 (44), 107 (68), 91 (100), 79 (68), 67 (83), 55 (59).
b. 1-((1S,5R)-6,6-Dimethylbicyclo[3.1.1]hept-2-en-2-yl)pent-4-en-1-ol (4.1 g, 20 mmol) and benzaldehyde (8.4 g, 82 mmol) according to the procedure in example 1 (step c) and gave 1-((1*S*,5*R*)-6,6-dimethylbicyclo[3.1.1]hept-2-en-2-yl)pent-4-en-1-one as colorless liquid (16 mmol, 3.3 g, 81% yield).
   ¹H NMR (400 MHz, CDCl₃) δ 6.75 - 6.61 (m, 1H), 5.80 - 5.71 (m, 1H), 5.03 - 4.84 (m, 2H), 2.88 (dt, *J* = 1.0, 5.6 Hz, 1H), 2.77 - 2.59 (m, 2H), 2.50 - 2.21 (m, 5H), 2.12 - 1.99 (m, 1H), 1.25 (s, 3H), 0.95 (d, *J* = 9.0 Hz, 1H), 0.66 (s, 3H) ppm. ¹³C NMR (101 MHz, CDCl₃) 198.5, 149.1, 137.5, 136.5, 115.0, 40.2, 39.5, 37.3, 36.1, 32.5, 31.1, 28.9, 25.8, 20.8 ppm. GC/MS (EI): m/z (%): 204 (1) [*M*⁺], 189 (2), 161 (18), 105 (100), 91 (24), 55 (59).

Odor description: green-galbanum, fruity-pineapple, metallic.

### Example 4: 1-((1S,5S)-6,6-Dimethylbicyclo[3.1.1]heptan-2-yl)pent-4-en-1-one

a. To a solution of (1*R*)-(-)-Myrtenal (20.0 g, 133 mmol) in EA (150 mL) was added 10% Pd/C (1.4 g) at room temperature, and the result suspension was hydrogenated at 1 atm at room temperature (rt) for 16 h. Purify the product by kugelrohr distillation and gave (1*S*,5*S*)-6,6-dimethylbicyclo[3.1.1]heptane-2-carbaldehyde (20.1 g, 132 mmol, yield: 99%) as colorless liquid.
b. (1*S*,5*S*)-6,6-Dimethylbicyclo[3.1.1]heptane-2-carbaldehyde (15.0 g, 99 mmol) reacted with but-3-en-1-ylmagnesium chloride (128 mL, 128 mmol, 1.0 M) according to the procedure in example 1 (step b) and 1-((1*S*,5*S*)-6,6-dimethylbicyclo[3.1.1]heptan-2-yl)pent-4-en-1-ol was obtained as light yellow liquid (13.6 g, 65 mmol, yield: 66%).
   GC/MS (EI): m/z (%): 208 (1) [*M*⁺], 193(6), 179 (2), 139 (14), 105 (15), 82 (81), 67 (100), 55 (86).
c. To a solution of 1-((1*S,*5*S*)-6,6-dimethylbicyclo[3.1.1]heptan-2-yl)pent-4-en-1-ol (10.0 g, 48 mmol) in DCM (200 mL) was added pyridinium chlorochromate (11.4 g, 53 mmol) portionwisely at 10°C and stirred at rt for 3 h. The reaction was quenched by adding hexane (300 mL), and the suspension was filtered through a short silica gel pad, the solvent of the filtrate was removed to get a crude oil. It was purified via silica gel chromatography (PE:MTBE=96:4) to give 1-((1*S*,5*S*)-6,6-dimethylbicyclo[3.1.1]heptan-2-yl)pent-4-en-1-one (1.9 g, 9.2 mmol, yield: 19%) as colorless liquid.
   ¹H NMR (400 MHz, CDCl₃) δ 5.91 - 5.64 (m, 1H), 5.05 - 4.95 (m, 2H), 2.81 - 2.79 (m, 1H), 2.57 - 2.25 (m, 7H), 2.00 - 1.70 (m, 4H), 1.25 - 1.15 (m, 4H), 0.72 (s, 3H) ppm. ¹³C NMR (101 MHz, CDCl₃) δ 212.0, 137.5, 115.0, 53.1, 43.0, 40.7, 39.2, 38.9, 30.1, 28.1, 27.3, 24.9, 22.0, 13.8 ppm. GC/MS (EI): m/z (%): 206 (1) [*M*⁺], 191 (5), 163 (7), 151 (11), 123 (46), 95 (30), 67 (43), 55 (100).

Odor description: fruity-pineapple, fatty, green-galbanum.

### Example 5: Allyl 6,6-dimethylbicyclo[3.1.1]hept-2-ene-2-carboxylate

a. To a solution of myrtenal (6,6-dimethylbicyclo[3.1.1]hept-2-ene-2-carbaldehyde, 3.0 g, 20 mmol), 2-methylbut-2-ene (7.0 g, 100 mmol) and 2-methylpropan-2-ol (7.4 g, 100 mmol) in water (100 mL) was added sodium chlorite (5.4 g, 60 mmol) and potassium phosphate (13.6 g, 100 mmol) in water (200 mL) slowly at 10°C, and the mixture was stirred for 16 h at rt. The reaction mixture was extracted with MTBE (100 mL*3) and washed with NaHCO₃ solution and brine, and dried over MgSO₄. The solvent was removed and the obtained crude oil was purified by distillation and 6,6-dimethylbicyclo[3.1.1]hept-2-ene-2-carboxylic acid (3.0 g, 18 mmol, yield: 90%) was obtained as colorless liquid.
b. To a solution of 6,6-dimethylbicyclo[3.1.1]hept-2-ene-2-carboxylic acid (1.5 g, 9.0 mmol) and potassium carbonate (1.6 g, 11.7 mmol) in DMF (25 mL) was added 3-bromoprop-1-ene (1.2 g, 9.9 mmol) and stirred for another 16 h at rt. The reaction was extracted by MTBE (50 mL*2) and dried over MgSO₄. The concentrated crude oil was purified via silica gel chromatography (PE:MTBE=97:3) and gave allyl 6,6-dimethylbicyclo[3.1.1]hept-2-ene-2-carboxylate (1.9 g, 8.2 mmol, yield: 91%) as colorless liquid.
   ¹H NMR (400 MHz, CDCl₃) δ 6.97 - 6.78 (m, 1H), 6.01 - 5.92 (m, 1H), 5.41 - 5.12 (m, 2H), 4.63 (d, *J =* 5.6 Hz, 2H), 2.88 - 2.78 (m, 1H), 2.55 - 2.33 (m, 3H), 2.14 - 2.11 (m, 1H), 1.33 (s, 3H), 1.12 (d, *J* = 9.1 Hz, 1H), 0.80 (s, 3H) ppm. ¹³C NMR (101 MHz, CDCl₃) δ 165.8, 140.1, 136.7, 132.6, 117.7, 64.9, 41.2, 40.3, 37.7, 32.2, 31.3, 25.9, 20.9 ppm. GC/MS (EI): m/z (%): 206 (1) [*M*⁺], 191 (1), 165 (10), 149 (9), 105 (100), 91 (46), 79 (38).

Odor description: aromatic, resinous, coniferous, metallic, fatty, fruity-pineapple, green-galbanum.

### Example 6: Allyl (1S,5R)-6,6-dimethylbicyclo[3.1.1]hept-2-ene-2-carboxylate

a. (1*S*,5*R*)-6,6-Dimethylbicyclo[3.1.1]hept-2-ene-2-carboxylic acid was obtained from the reaction of (1*S*,5*R*)-6,6-dimethylbicyclo[3.1.1]hept-2-ene-2-carbaldehyde (2.5 g, 17 mmol), 2-methylbut-2-ene (5.8 g, 83 mmol), 2-methylpropan-2-ol (6.2 g, 83 mmol) and sodium chlorite (4.6 g, 51 mmol) according to the procedure in example 5 (step a) as colorless liquid (16 mmol, 2.6 g, 94% yield).
b. According to the procedure of example 5 (step b), (1S,5R)-6,6-dimethylbicyclo[3.1.1]hept-2-ene-2-carboxylic acid (1.5 g, 9.0 mmol) reacted with 3-bromoprop-1-ene (1.2 g, 9.9 mmol) and got allyl (1*S*,5*R*)-6,6-dimethylbicyclo[3.1.1]hept-2-ene-2-carboxylate as colorless liquid (7.8 mmol, 1.6 g, 86% yield).

¹H NMR (400 MHz, CDCl₃) δ 6.91 - 6.80 (m, 1H), 6.01 - 5.91 (m, 1H), 5.37 - 5.14 (m, 2H), 4.63 (d, *J =* 5.6 Hz, 2H), 2.82 - 2.80 (m, 1H), 2.51 - 2.32 (m, 3H), 2.17 - 2.07 (m, 1H), 1.33 (s, 3H), 1.12 (d, *J* = 9.1 Hz, 1H), 0.80 (s, 3H) ppm. ¹³C NMR (101 MHz, CDCl₃) δ 165.8, 140.1, 136.7, 132.6, 117.7, 64.9, 41.2, 40.3, 37.7, 32.2, 31.3, 25.9, 20.9 ppm. GC/MS (EI): m/z (%): 206 (1) [*M*⁺], 191 (1), 165 (7), 105 (100), 91 (51), 79 (45).

Odor description: green-galbanum, metallic, carrot, garlic, fatty, fruity-pineapple.

### Example 7: Allyl (1R,5S)-6,6-dimethylbicyclo[3.1.1]hept-2-ene-2-carboxylate

a. (1*R*,5*S*)-6,6-Dimethylbicyclo[3.1.1]hept-2-ene-2-carboxylic acid was obtained from the reaction of (1*R*,5*S*)-6,6-dimethylbicyclo[3.1.1]hept-2-ene-2-carbaldehyde (20.0 g, 133 mmol), 2-methylbut-2-ene (46.7 g, 666 mmol) , 2-methylpropan-2-ol (49.3 g, 666 mmol) and sodium chlorite (36.0 g, 399 mmol) according to the procedure of example 5 (step 1) as colorless liquid (130mmol, 21.6 g, 97% yield).
b. (1*R*,5*S*)-6,6-Dimethylbicyclo[3.1.1]hept-2-ene-2-carboxylic acid (5.0 g, 30 mmol) reacted with 3-bromoprop-1-ene (4.0 g, 33 mmol) according to the procedure of example 5 (step b) and gave allyl (1*R*,5*S*)-6,6-dimethylbicyclo[3.1.1]hept-2-ene-2-carboxylate as colorless liquid (27 mmol, 5.6 g, 90% yield).

¹H NMR (400 MHz, CDCl₃) δ 6.88 - 6.84 (m, 1H), 6.01 - 5.91 (m, 1H), 5.35 - 5.21 (m, 2H), 4.63 (d, *J =* 5.5 Hz, 2H), 2.83 - 2.80 (m, 1H), 2.52 - 2.33 (m, 3H), 2.17 - 2.03 (m, 1H), 1.33 (s, 3H), 1.12 (d, *J* = 9.1 Hz, 1H), 0.80 (s, 3H) ppm. ¹³C NMR (101 MHz, CDCl₃) δ 165.8, 140.0, 136.8, 132.6, 117.7, 64.9, 41.2, 40.3, 37.7, 32.2, 31.3, 25.9, 20.9 ppm. GC/MS (EI): m/z (%): 206 (1) [*M*⁺], 191 (1), 165 (7), 105 (100), 91 (53), 79 (46).

Odor description: green, metallic, galbanum, aromatic.

### Example 8: 1-(7,7-Dimethylbicyclo[4.1.0]hept-3-en-3-yl)pent-4-en-1-one

a. To a solution of diisopropylamine (47.9 g, 473 mmol) in THF (200 ml) was added n-butyllithium (296 mL, 473 mmol) slowly at -78°C and stirred for 30 min under argon atmosphere at rt. Then 3-carene epoxide (3,8,8-trimethyl-4-oxatricyclo[5.1.0.0^{3,5}]octane, 60.0 g, 394 mmol) in THF (200 mL) was added slowly at -78°C and stirred at rt for 5 h. Saturated NH₄Cl (100 mL) was added, and the mixture was extracted with MTBE (200 mL*3), the organic layer was washed with NaHCO₃ solution and brine, and then dried over MgSO₄. The solvent was removed and the crude oil was distilled to give 3(10)-caren-4-ol (7,7-dimethyl-4-methylenebicyclo[4.1.0]heptan-3-ol, 56.6 g, 372 mmol, 94% yield) as colorless liquid.
b. To a solution of 3(10)-caren-4-ol (7,7-dimethyl-4-methylenebicyclo[4.1.0]heptan-3-ol, 15.0 g, 99 mmol) in toluene (50 mL) was added (((oxovanadio)oxy)triphenyl-l5-silyl)((triphenyl(((triphenyl(l1-phosphaneyl)-l5-silyl)oxy)-l2-phosphaneyl)-l5-silyl)oxy)-l2-phosphane (5.8 g, 5.9 mmol) at 10°C and stirred at 100°C for 6 h. Cool down to r.t. and water (50 mL) was added, it was then extracted with MTBE (100 mL*3), the organic layer was dried over MgSO₄. Concentrate the organic layer and the result crude oil was purified via silica gel chromatography (PE:MTBE=9:1) and gave 3-caren-10-ol ((7,7-dimethylbicyclo[4.1.0]hept-3-en-3-yl)methanol, 6.8 g, 45 mmol, yield: 45%) as light yellow liquid.
c. A solution of 3-caren-10-ol ((7,7-dimethylbicyclo[4.1.0]hept-3-en-3-yl)methanol, 5.0 g, 33 mmol) in DCM (100 mL) was added pyridinium chlorochromate (9.2 g, 43 mmol) and stirred at rt. for 5 h. Hexane (200mL) was added, and the result suspension was filtered. The filtrate was concentrated and the result crude oil was purified via silica gel chromatography (PE:MTBE=97:3) and gave 7,7-dimethylbicyclo[4.1.0]hept-3-ene-3-carbaldehyde (1.3 g, 8.7 mmol, yield: 26%) as colorless liquid.
d. According to the procedure in example 1 (step b), but-3-en-1-ylmagnesium chloride (17.5 mL, 8.7 mmol, 0.5 M) reacted with 7,7-dimethylbicyclo[4.1.0]hept-3-ene-3-carbaldehyde (1.0 g, 6.7 mmol) and gave 1-(7,7-dimethylbicyclo[4.1.0]hept-3-en-3-yl)pent-4-en-1-ol (1.0 g, 4.8 mmol, yield: 73%) as light yellow liquid.
   ¹H NMR (400 MHz, CDCl₃) δ 5.88 - 5.71 (m, 1H), 5.59 - 5.53 (m, 1H), 5.10 - 4.88 (m, 2H), 4.03 - 3.95(m, 1H), 2.47 - 1.78 (m, 5H), 1.68 - 1.59 (m, 2H), 1.43 - 1.25 (m, 2H), 1.04, 1.04 (s, 3H), 0.93 - 0.62 (m, 5H) ppm. ¹³C NMR (101 MHz, CDCl₃) δ 138.5, 138.4, 137.6, 137.5, 122.4, 121.6, 114.7, 114.6, 76.5, 76.0, 34.2, 33.9, 30.2, 30.1, 28.4, 28.3, 20.6, 20.5, 18.6, 17.8, 17.7, 17.6, 17.4, 17.2, 17.0, 13.4, 13.4 ppm.
e. According to the procedure in example 1 (step b), 1-(7,7-dimethylbicyclo[4.1.0]hept-3-en-3-yl)pent-4-en-1-ol (0.9 g, 4.4 mmol) reacted wth aluminium tri-sec-butoxide (0.3 g, 1.3 mmol) and benzaldehyde (1.9 g, 17.0 mmol) and gave 1-(7,7-dimethylbicyclo[4.1.0]hept-3-en-3-yl)pent-4-en-1-one (0.50 g, 2.5 mmol, 57% yield) as colorless liquid.
   ¹H NMR (400 MHz, CDCl₃) δ 6.88 - 6.72 (m, 1H), 5.86 - 5.77 (m, 1H), 5.04 - 4.94 (m, 2H), 2.74 - 2.57 (m, 3H), 2.57 - 2.46 (m, 1H), 2.40 - 2.28 (m, 2H), 2.27 - 2.14 (m, 2H), 1.05 (s, 3H), 0.88 - 0.78 (m, 1H), 0.74 - 0.66 (m, 4H) ppm. ¹³C NMR (101 MHz, CDCl₃) δ 200.2, 138.5, 137.7, 137.2, 114.9, 36.5, 28.7, 28.1, 22.1, 18.1, 17.8, 17.5, 16.6, 13.5 ppm. GC/MS (EI): m/z (%): 204 (6) [*M*⁺], 189 (5), 175 (2), 161 (28), 105 (100), 55 (56).

Odor description: green, fatty, metallic, galbanum, aromatic, coniferous, fir needle.

### Example 8.1: 1-((1S,6R)-7,7-Dimethylbicyclo[4.1.0]hept-3-en-3-yl)pent-4-en-1-one

a. To a solution of (1*S*,6*R*)-7,7-dimethylbicyclo[4.1.0]hept-3-ene-3-carbaldehyde (7.0 g, 47 mmol) in THF (25 mL) was added but-3-en-1-ylmagnesium chloride (121 mL, 61 mmol, 0.5 M) in THF (50 mL) at 10 °C and stirred at rt for 5 h. A solution of saturated NH₄Cl (100 mL) aqueous solution was added. The mixture was extracted with EA (200 mL*3). The combined organic layer was washed with saturated NaHCO₃ solution (100mL) and brine (100 mL) and then dried over MgSO₄ and filtered. The solvent was removed by rotary evaporate and the result crude oil was purified via silica gel chromatography (heptane:MTBE=4:1) to give 1-((1*S*,6*R*)-7,7-dimethylbicyclo[4.1.0]hept-3-en-3-yl)pent-4-en-1-ol (7.1 g, 34 mmol, yield: 74%) as a colorless liquid.
   ¹H NMR (400 MHz, CDCl₃, mixture of isomers) *δ* 5.87 - 5.77 (m, 1H), 5.58 - 5.51 (m, 1H), 5.08 - 4.90 (m, 2H), 4.01 - 3.95 (m, 1H), 2.42 - 1.26 (m, 9H), 1.04 - 0.64 (m, 8H) ppm. ¹³C NMR (101 MHz, CDCl₃, mixture of isomers) *δ* 138.5, 138.4, 137.6, 137.5, 122.3, 121.5, 114.7, 114.6, 76.4, 75.9, 34.2, 33.9, 30.2, 30.1, 28.4, 28.3, 20.5, 20.4, 18.6, 17.8, 17.7, 17.4, 17.2, 17.0, 13.4, 13.3 ppm.
   GC/MS (EI): *m*/*z* (%): 206 (12) [*M*⁺], 191 (10), 173 (9), 163 (29), 145 (40), 119 (21), 105 (77), 91 (99), 79 (100), 67 (70), 55 (74).
b. A mixture of 1-((1*S*,6*R*)-7,7-dimethylbicyclo[4.1.0]hept-3-en-3-yl)pent-4-en-1-ol (7.0 g, 34 mmol), aluminium tri-sec-butoxide (2.5 g, 10 mmol) and acetic acid (204 mg, 3.4 mmol) in MTBE (100 mL) was stirred at rt for 10 min. 4-Nitrobenzaldehyde (11.5 g, 76 mmol) was added and stirred at rt for 6 h. Water (50 mL) was added, and the result mixture was extracted with MTBE (200 mL*3). The combined organic layer was dried over MgSO₄, and filtered. The solvent was removed by rotary evaporate and the result crude oil was purified via silica gel chromatography (heptane:MTBE=96:4) to give 1-((1*S*,6*R*)-7,7-dimethylbicyclo[4.1.0]hept-3-en-3-yl)pent-4-en-1-one (6.6 g, 32 mmol, yield: 95%) as a colorless liquid.
   ¹H NMR (400 MHz, CDCl₃) δ 6.83 -6.81 (m, 1H), 5.86 -5.77 (m, 1H), 5.07 - 4.95 (m, 2H), 2.75 - 2.46 (m, 4H), 2.37 - 3.31 (m, 2H), 2.27 - 2.12 (m, 2H), 1.05 (s, 3H), 0.85 - 0.69 (m, 2H), 0.69 (s, 3H) ppm. ¹³C NMR (101 MHz, CDCl₃) δ 200.2, 138.5, 137.6, 137.2, 114.9, 36.4, 28.7, 28.1, 22.1, 18.1, 17.8, 17.5, 16.6, 13.5 ppm.
   GC/MS (EI): *m*/*z* (%): 204 (6) [*M*⁺], 189 (4), 161 (27), 149 (17), 121 (31), 105 (100), 77 (35), 55 (59).

Odor description: green, galbanum, fatty, fruity

### Example 9: Allyl 7,7-dimethylbicyclo[4.1.0]hept-3-ene-3-carboxylate

a. 7,7-Dimethylbicyclo[4.1.0]hept-3-ene-3-carboxylic acid was obtained from the reaction of 7,7-dimethylbicyclo[4.1.0]hept-3-ene-3-carbaldehyde (4.0 g, 17.3 mmol), 2-methylbut-2-ene (6.1 g, 86.5 mmol), 2-methylpropan-2-ol (6.4 g, 86.5 mmol) and sodium chlorite (4.7 g, 51.9 mmol) according to the procedure of example 5 (step 1) as white solid (2.1 g, 11.0 mmol, purity: 90%, yield: 66%).
   ¹³C NMR (101 MHz, CDCl₃) δ 171.7, 139.9, 126.8, 27.1, 21.0, 17.7, 16.7, 16.6, 15.5, 12.4 ppm.
b. 7,7-Dimethylbicyclo[4.1.0]hept-3-ene-3-carboxylic acid (2.1 g, 11.0 mmol, purity: 90%) reacted with 3-bromoprop-1-ene (2.1 g, 17.1 mmol) according to the procedure of example 5 (step b) and gave allyl 7,7-dimethylbicyclo[4.1.0]hept-3-ene-3-carboxylate (1.6 g, 7.8 mmol, yield: 68%) as colorless liquid.
   ¹H NMR (400 MHz, CDCl₃) δ 7.04 - 6.77 (m, 1H), 5.98 - 5.90 (m, 1H), 5.39 - 5.17 (m, 2H), 4.64 - 4.62 (m, 2H), 2.69 - 2.53 (m, 2H), 2.28 - 2.09 (m, 2H), 1.06 (s, 3H), 0.87 - 0.64 (m, 5H) ppm. ¹³C NMR (101 MHz, CDCl₃) δ 167.0, 138.5, 132.5, 128.2, 117.7, 64.9, 28.2, 21.8, 19.0, 17.8, 17.5, 16.6, 13.4 ppm.

Odor description: green, fruity-pineapple, galbanum.

### Example 9.1: Allyl (1S,6R)-7,7-dimethylbicyclo[4.1.0]hept-3-ene-3-carboxylate

a. To a solution of (1S,6R)-3,7,7-trimethylbicyclo[4.1.0]hept-3-ene (50.0 g, 367 mmol) in CHCl₃ (400 mL) was added *m*-CPBA (80.5 g, 85% Wt, 396 mmol) slowly at 23-27°C and stirred at rt overnight. Heptane (200 mL) was added and the solid was filtered off. 500 mL of EA was added and the result mixture was washed with NaHSO₃ (200 mL*3), saturated NaHCO₃ aqueous solution (200 mL*3) and brine (200 mL). The result organic layer dried over MgSO₄ and then filtered. The solvent was removed by rotary evaporation. The crude product was purified by silica gel chromatography (heptane: MTBE=94:6) to give (1S,7R)-3,8,8-trimethyl-4-oxatricyclo[5.1.0.0^{3,5}]octane (51.6 g, 339 mmol, yield: 92%) as a colorless liquid.
   ¹H NMR (400 MHz, CDCl₃) δ 2.84 - 2.79 (m, 1H), 2.33 - 2.11 (m, 2H), 1.66 - 1.47 (m, 2H), 1.26 (s, 3H), 1.01 (s, 3H), 0.73 (s, 3H), 0.57 - 0.41 (m, 2H) ppm. ¹³C NMR (101 MHz, CDCl₃) δ 58.1, 55.8, 27.7, 23.3, 23.1, 19.2, 16.0, 15.9, 14.6, 13.8 ppm.
b. To a solution of 2,2,6,6-tetramethylpiperidine (50.1 g, 355 mmol) in THF (500 mL) was added n-butyllithium (222 mL, 1.6 M, 355 mmol) slowly at 0°C and stirred at rt under argon for 30 min. Diethylaluminumchloride (359 mL, 1.0 M, 358 mmol) was added slowly at 0°C and stirred at rt for 30 min. Then (1*S*,7*R*)-3,8,8-trimethyl-4-oxatricyclo[5.1.0.0^{3,5}]octane (30.0 g, 177 mmol) in THF (50 mL) was added slowly at 0°C and stirred at rt for 16 h. The mixture was quenched by adding 500 mL of 10% HCl aqueous solution, and the result mixture was extracted with MTBE (300 mL*3). The combined organic layer was washed with saturated NaHCO₃ aqueous solution and brine, and then dried over MgSO₄ and filtered. The solvent was removed and the crude oil was distilled via kugelrohr distillation (0.11 mbar, 120°C) to give (1*R*,6*S*)-7,7-dimethyl-4-methylenebicyclo[4.1.0]heptan-3-ol with 85% purity as colorless liquid (29.2 g, 160 mmol, 92% yield).
c. To a solution of (1*R*,6*S*)-7,7-dimethyl-4-methylenebicyclo[4.1.0]heptan-3-ol (16.0 g, 85% Wt, 89 mmol) in toluene (300 mL) was added tris(triphenylsiloxy)vanadium oxide (5.3 g, 5.4 mmol) at 10°C and stirred at 110°C for 6 h. Water (50 mL) and MTBE (200 mL) were added, and the mixture was extracted with MTBE (200 mL*3). The combined organic layer was washed with saturated NaHCO₃ aqueous solution (200 mL) and brine (200 mL), and dried over MgSO₄ and filtered. The solvent was removed and the result crude oil was purified via silica gel chromatography (heptane:MTBE=9:1) to give ((1S,6R)-7,7-dimethylbicyclo[4.1.0]hept-3-en-3-yl)methanol (12.1 g, 80 mmol, yield: 89%) as a colorless liquid.
   ¹H NMR (400 MHz, CDCl₃) δ 5.60 - 5.50 (m, 1H), 3.95 (s, 2H), 2.46 - 2.25 (m, 2H), 2.01 - 1.88 (m, 2H), 1.65 (s, 1H), 1.04 (s, 3H), 0.80 - 0.70 (m, 4H), 0.70 - 0.59 (m, 1H) ppm. ¹³C NMR (101 MHz, CDCl₃) δ 135.3, 121.7, 67.7, 28.3, 20.5, 20.4, 17.8, 17.1, 16.9, 13.3 ppm.
d. A mixture of ((1*S*,6*R*)-7,7-dimethylbicyclo[4.1.0]hept-3-en-3-yl)methanol (12.0 g, 79 mmol), sodium ethanolate (8.4 g, 102 mmol) and MgSO₄ (10 g) in DCM (200 mL) was stirred at 10°C for 10 min. Then a mixture of pyridinium chlorochromate (22.1 g, 102 mmol) and silica gel (30 g) was added slowly and stirred at rt for 6 h. MTBE (200 mL) was added, and the mixture was filtered through short silica gel column. The solvent was removed and the result crude oil was purified via silica gel chromatography (heptane:MTBE=97:3) to give (1*S*,6*R*)-7,7-dimethylbicyclo[4.1.0]hept-3-ene-3-carbaldehyde (8.6 g, 57mmol, yield: 83%) as a colorless liquid.
   ¹H NMR (400 MHz, CDCl₃) *δ* 9.40 (s, 1H), 6.72 - 6.71 (m, 1H), 2.77 - 2.18 (m, 4H), 1.07 (s, 3H), 0.83 - 0.70 (m, 5H) ppm. ¹³C NMR (101 MHz, CDCl₃) δ 194.1, 150.6, 139.9, 28.2, 22.5, 17.7, 17.6, 17.1, 16.6, 13.5 ppm.
   GC/MS (EI): *m*/*z* (%): 150 (4) [*M*⁺], 135 (11), 121 (19), 107 (100), 91 (33), 79 (79).
e. To a mixture of (1*S*,6*R*)-7,7-dimethylbicyclo[4.1.0]hept-3-ene-3-carbaldehyde (25.0 g, 166 mmol), KH₂PO₄ (113 g, 832 mmol), 2-methylbut-2-ene (58.4 g, 832 mmol) and 2-methylpropan-2-ol (61.7 g, 832 mmol ) in water (200 mL) was added a solution of sodium chlorite (45.2 g, 499 mmol) in water (100 mL) at 10°C and stirred at rt for 16 h. Saturated NH₄Cl (300 mL) solution was added. The mixture was extracted with EA (100 mL*3). The combined organic layer was washed with saturated NaHCO₃ aqueous solution and brine and then dried over MgSO₄ and filtered. The solvent was removed and the crude oil was purified via silica gel chromatography (heptane:MTBE=9:1) to give (1*S*,6*R*)-7,7-dimethylbicyclo[4.1.0]hept-3-ene-3-carboxylic acid (25.2 g, 152 mmol, yield: 91%) as a colorless liquid.
   ¹H NMR (400 MHz, CDCl₃) *δ* 7.13 - 6.96 (m, 1H), 2.65 - 2.47 (m, 2H), 2.28 - 2.10 (m, 2H), 1.06 (s, 3H), 0.87 - 0.78 (m, 1H), 0.76 - 0.65 (m, 4H) ppm. ¹³C NMR (101 MHz, CDCl₃) δ 172.6, 140.9, 127.7, 28.2, 22.1, 18.7, 17.7, 17.6, 16.5, 13.4 ppm.
   GC/MS (EI): m/z (%): 166 (16) [*M*⁺], 123 (87), 105 (52), 91 (32), 79 (100).
f. A suspension of (1*S*,6*R*)-7,7-dimethylbicyclo[4.1.0]hept-3-ene-3-carboxylic acid (25.0 g, 150 mmol), 3-bromoprop-1-ene (27.3 g, 226 mmol) and potassium carbonate (41.6 g, 301mmol) in DMF (200 mL) was stirred at 80°C for 6 h. EA (100 mL) was added and filtered. The solvent was removed by rotary evaporation and the result crude oil was purified via silica gel chromatography (heptane:MTBE=98:2) to give allyl (1*S*,6*R*)-7,7-dimethylbicyclo[4.1.0]hept-3-ene-3-carboxylate (26.6 g, 129 mmol, yield: 86%) as a colorless liquid.
   ¹H NMR (400 MHz, CDCl₃) *δ* 6.99 - 6.87 (m, 1H), 6.00 - 5.86 (m, 1H), 5.35 - 5.21 (m, 2H), 4.64 - 4.62 (m, 2H), 2.65 - 2.48 (m, 2H), 2.28 - 2.09 (m, 2H), 1.06 (s, 3H), 0.88 - 0.65 (m, 5H) ppm. ¹³C NMR (101 MHz, CDCl₃) *δ* 167.0, 138.5, 132.5, 128.2, 117.7, 64.9, 28.2, 21.8, 19.0, 17.7, 17.5, 16.5, 13.4 ppm.
   GC/MS (EI): *m*/*z* (%): 206 (1) [*M*⁺], 191 (1), 165 (22), 123 (100), 105 (57), 91 (36), 79 (48).

Odor description: green, galbanum, metallic, fatty.

### Example 10: 1-(4,7,7-Trimethylbicyclo[4.1.0]hept-3-en-3-yl)pent-4-en-1-one

a. To a solution of acetyl carene (1-(4,7,7-trimethylbicyclo[4.1.0]hept-4-en-3-yl)ethan-1-one, 10.0 g, 56 mmol) in tetrahydrofuran (200 mL) was added DBU (25.6 g, 168 mmol) at rt and stirred for 12 h at rt. A solution of NH₄Cl (100 mL) was added and the mixture was extracted with MTBE (150 mL*3), and the combined organic layer was dried over MgSO₄. The solvent was removed and the crude oil was purified by distillation. 1-(4,7,7-Trimethylbicyclo[4.1.0]hept-3-en-3-yl)ethan-1-one (1.7 g, 9.0 mmol, 16% yield) was obtained as colorless liquid.
b. To a solution of 1-(4,7,7-trimethylbicyclo[4.1.0]hept-3-en-3-yl)ethan-1-one (3.2 g, 18 mmol) in Tetrahydrofuran (100 mL) was added lithium bis(trimethylsilyl)amide solution (4.0 mL, 20 mmol) slowly at -20°C in 30 min and stirred at -20°C for another 30 min under argon. 3-Bromoprop-1-ene (2.1 g, 17 mmol) was added at 0°C and stirred at r.t. for 12 h. A solution of saturated NH₄Cl (100 mL) was added, and the mixture was extracted with MTBE (100 mL*3), the combined organic layer was dried over MgSO₄. The solvent was removed and the residue was purified via silica gel chromatography (PE:MTBE=96:4) to give 1-(4,7,7-trimethylbicyclo[4.1.0]hept-3-en-3-yl)pent-4-en-1-one (0.31 g, 1.4 mmol, yield: 8%) as colorless liquid.

¹H NMR (400 MHz, CDCl₃) δ 5.88 - 5.78 (m, 1H), 5.10 - 4.89 (m, 2H), 2.66 - 2.30 (m, 6H), 2.20 - 1.94 (m, 2H), 1.73 (s, 3H), 1.04 (s, 3H), 0.82 - 0.66 (m, 5H) ppm. ¹³C NMR (101 MHz, CDCl₃) δ 207.2, 137.5, 136.1, 131.7, 115.1, 40.8, 28.0, 27.9, 27.9, 21.9, 21.1, 17.9, 17.2, 17.0, 13.5 ppm. GC/MS (EI): m/z (%): 218 (64) [*M*⁺], 203 (42), 175 (32), 163 (67), 147 (74), 119 (100), 91 (87), 55 (96).

Odor description: green, rubbery, metallic, galbanum, aromatic.

### Example 11: Fragrance accord

| Ingredient | parts by weight |
|---|---|
| ALDEHYDE C 12 MNA (2-methylundecanal) | 25 |
| AMBROFIX^{™} (3A,6,6,9a-tetramethyldodecahydronaphtho[2,1-b]furan) | 5 |
| BENZYL ACETONE (4-phenyl-2-butanone) | 15 |
| COUMARIN pure crystals (2H-1-BENZOPYRAN-2-ONE) | 5 |
| DELTA DAMASCONE | 3 |
| DIHYDRO MYRCENOL (2,6-dimethyl-7-octen-2-ol) | 150 |
| DIPROPYLENE GLYCOL | 48 |
| GALBANONE 10 TM (1-(5,5-dimethylcyclohex-1-en-1-yl)pent-4-en-1-one) | 15 |
| GERANIOL (3,7-dimethyl-2,6-octadien-1-ol) | 60 |
| HEDIONE^{™}(methyl 3-oxo-2-pentylcyclopentaneacetate) | 85 |
| HEXYL SALICYLATE | 200 |
| ISO E SUPER ^{™} (2-acetyl-1,2,3,4,5,6,7,8-octahydro-2,3,8,8-tetra-methylnaphtalene) | 86 |
| ISOEUGENOL (2-methoxy-4-propenylphenol) | 5 |
| JAVANOL^{™ 1}) | 0.5 |
| LAVANDIN GROSSO OIL FRANCE ORPUR | 20 |
| LILIAL^{™} (2-methyl-3-(4-(1,1-dimethylethyl)phenyl)propanal) | 125 |
| METHYL SALICYLATE | 1.5 |
| PEONILE^{™} (2-cyclohexylidene-2-phenylacetonitrile) | 60 |
| PHENYL ETHYL ACETATE | 60 |
| ROSE OXIDE (4-methyl-2-(2-methyl-1-propenyl)tetrahydro-2h-pyran) | 3 |
| TRICYCLAL (2,4-dimethyl-3-cyclohexene-1-carbaldehyde) | 8 |
| UNDECAVERTOL (4-methyl-3-decen-5-ol) | 20 |
| Total | 1000 |

### 1) (1-methyl-2-(1,2,2-trimethylbicyclo(3.1.0)-hex-3-ylmethyl)cyclopropyl)methanol

The fragrance accord above is a classic fresh, clean accord, e.g., suitable to be admixed to a fabric heavy duty liquid detergent (HDLD). The fragrance accord might be admixed, e.g., at about 0.6wt% or about 0.8wt% to the HDLD base.

By replacement of 15 parts DPG with 15 parts of 1-(6,6-dimethylbicyclo[3.1.1]hept-2-en-2-yl)pent-4-en-1-one (e.g., 1-((1S,5R)-6,6-dimethylbicyclo[3.1.1]hept-2-en-2-yl)pent-4-en-1-one, the fragrance performance is noticeably increased in use, especially in the wet phase assessment, with also a benefit on dry fabric after 24 hours. The addition of the compound of formula (I) delivers a classical fresh and clean green, galbanum pineapple character that combines very well with the accord and is fitting very well to the fresh and clean expectation of the consumer.

## Claims

1. A compound of formula (I) 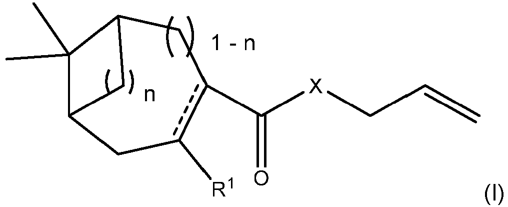 wherein
the dotted line together with the carbon-carbon bond forms a single bond or a double bond;
n is 1 or 0;
X is selected from -O- and -CH₂-; and
R' is hydrogen or methyl.

2. The compound according to claim 1 wherein the compound of formula (I) is a compound of formula (Ia) 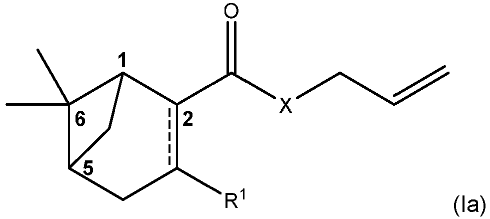 wherein
the dotted line together with the carbon-carbon bond forms a single bond or a double bond;
X is selected from -O- and -CH₂-; and
R' is hydrogen or methyl.

3. The compound according to claim 1 or claim 2 wherein the dotted line together with the carbon-carbon bond forms a double bond.

4. The compound according to claim 2 wherein the dotted line together with the carbon-carbon bond forms a double bond and wherein the relative configuration of the ring system is (1 S,5R).

5. The compound according to claim 1 wherein the compound is selected from the group consisting of 1-(6,6-dimethylbicyclo[3.1.1]hept-2-en-2-yl)pent-4-en-1-one, 1-(6,6-dimethylbicyclo[3.1.1]heptan-2-yl)pent-4-en-1-one, allyl 6,6-dimethylbicyclo[3.1.1]hept-2-ene-2-carboxylate, 1-(7,7-dimethylbicyclo[4.1.0]hept-3-en-3-yl)pent-4-en-1-one, allyl 7,7-dimethylbicyclo[4.1.0]hept-3-ene-3-carboxylate and 1-(4,7,7-trimethylbicyclo[4.1.0]hept-3-en-3-yl)pent-4-en-1-one.

6. A fragranced article comprising as odorant a compound of formula (I) as defined in claim 1 to 5, or a mixture thereof, and a consumer product base.

7. A fragranced article according to claim 6 wherein the consumer product bases is selected from fine fragrance, household products, laundry products, body care products, cosmetic products and air care products.

8. The use as fragrance of a compound of formula (I) as defined in claim 1 to 5.

9. A method of improving, enhancing or modifying a consumer product base by means of addition thereto of an olfactory acceptable amount of a compound of formula (I) as defined in claim 1.

## Patentansprüche

1. Verbindung der Formel (I) 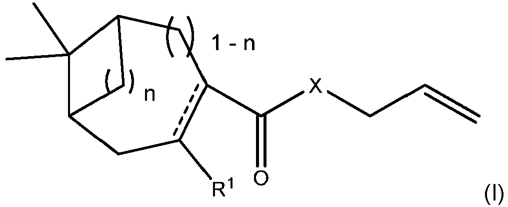 wobei
die gestrichelte Linie zusammen mit der Kohlenstoff-Kohlenstoff-Bindung eine Einfachbindung oder eine Doppelbindung bildet;
n für 1 oder 0 steht;
X aus -O- und -CH₂- ausgewählt ist; und
R¹ für Wasserstoff oder Methyl steht.

2. Verbindung nach Anspruch 1, wobei es sich bei der Verbindung der Formel (I) um eine Verbindung der Formel (Ia) 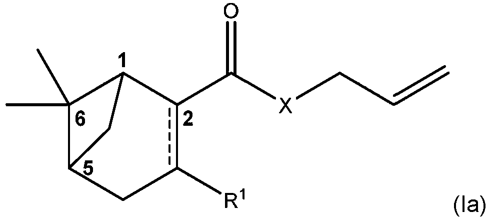 handelt, wobei
die gestrichelte Linie zusammen mit der Kohlenstoff-Kohlenstoff-Bindung eine Einfachbindung oder eine Doppelbindung bildet;
X aus -O- und -CH₂- ausgewählt ist; und
R¹ für Wasserstoff oder Methyl steht.

3. Verbindung nach Anspruch 1 oder Anspruch 2, wobei die gestrichelte Linie zusammen mit der Kohlenstoff-Kohlenstoff-Bindung eine Doppelbindung bildet.

4. Verbindung nach Anspruch 2, wobei die gestrichelte Linie zusammen mit der Kohlenstoff-Kohlenstoff-Bindung eine Doppelbindung bildet und wobei die relative Konfiguration des Ringsystems (1S,5R) ist.

5. Verbindung nach Anspruch 1, wobei die Verbindung aus der Gruppe bestehend aus 1-(6,6-Dimethylbicyclo[3.1.1]hept-2-en-2-yl)pent-4-en-1-on, 1-(6,6-Dimethylbicyclo[3.1.1]heptan-2-yl)pent-4-en-1-on, 6,6-Dimethylbicyclo[3.1.1]hept-2-en-2-carbonsäureallylester, 1-(7,7-Dimethylbicyclo[4.1.0]hept-3-en-3-yl)pent-4-en-1-on, 7,7-Dimethylbicyclo[4.1.0]hept-3-en-3-carbonsäureallylester und 1-(4,7,7-Trimethylbicyclo[4.1.0]hept-3-en-3-yl)pent-4-en-1-on ausgewählt ist.

6. Duftstoffhaltiger Artikel, umfassend eine Verbindung der Formel (I) gemäß einem der Ansprüche 1 bis 5 oder eine Mischung davon als Riechstoff und eine Konsumproduktbasis.

7. Duftstoffhaltiger Artikel nach Anspruch 6, wobei die Konsumproduktbasis aus Feinduftstoffen, Haushaltsprodukten, Waschmittelprodukten, Körperpflegeprodukten, Kosmetikprodukten und Luftbehandlungsprodukten ausgewählt ist.

8. Verwendung einer Verbindung der Formel (I) gemäß einem der Ansprüche 1 bis 5 als Duftstoff.

9. Verfahren zur Verbesserung, Verstärkung oder Modifizierung einer Konsumproduktbasis durch Zugabe einer olfaktorisch annehmbaren Menge einer Verbindung der Formel (I) gemäß Anspruch 1.

## Revendications

1. Composé de formule (I) 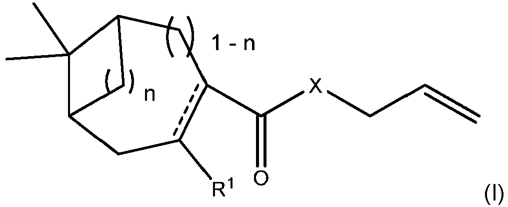 dans laquelle
la ligne en pointillés conjointement avec la liaison carbone-carbone forme une liaison simple ou une double liaison ;
n est 1 ou 0 ;
X est choisi parmi -O- et -CH₂- ; et
R¹ est hydrogène ou méthyle.

2. Composé selon la revendication 1, dans lequel le composé de formule (I) est un composé de formule (Ia) 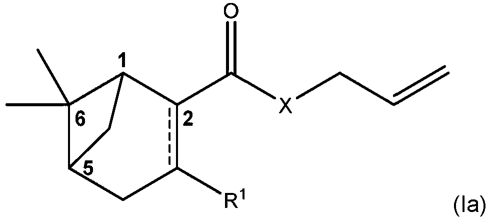 dans laquelle
la ligne en pointillés conjointement avec la liaison carbone-carbone forme une liaison simple ou une double liaison ;
X est choisi parmi -O- et -CH₂- ; et
R¹ est hydrogène ou méthyle.

3. Composé selon la revendication 1 ou la revendication 2, dans lequel la ligne en pointillés, conjointement avec la liaison carbone-carbone, forme une double liaison.

4. Composé selon la revendication 2, dans lequel la ligne en pointillés conjointement avec la liaison carbone-carbone forme une double liaison et dans lequel la configuration relative du système cyclique est (1S,5R).

5. Composé selon la revendication 1, dans lequel le composé est choisi dans le groupe constitué par 1-(6,6-diméthylbicyclo[3.1.1]hept-2-én-2-yl)pent-4-én-1-one, 1-(6,6-diméthylbicyclo[3.1.1]heptan-2-yl)pent-4-én-1-one, 6,6-diméthylbicyclo[3.1.1]hept-2-ène-2-carboxylate d'allyle, 1-(7,7-diméthylbicyclo[4.1.0]hept-3-én-3-yl)pent-4-én-1-one, 7,7-diméthylbicyclo[4.1.0]hept-3-ène-3-carboxylate d'allyle et 1-(4,7,7-triméthylbicyclo[4.1.0]hept-3-én-3-yl)pent-4-én-1-one.

6. Article fragrancé comprenant comme substance odorante un composé de formule (I) tel que défini dans la revendication 1 à 5, ou un mélange correspondant, et une base de produit de consommation.

7. Article fragrancé selon la revendication 6, dans lequel les bases de produit de consommation sont choisies parmi une fragrance fine, des produits ménagers, des produits de lessive, des produits de soin du corps, des produits cosmétiques et des produits d'assainissement de l'air.

8. Utilisation comme fragrance d'un composé de formule (I) tel que défini dans la revendication 1 à 5.

9. Procédé d'amélioration, d'augmentation ou de modification d'une base de produit de consommation par addition à celle-ci d'une quantité acceptable sur le plan olfactif d'un composé de formule (I) tel que défini dans la revendication 1.
